# EUROPEAN PATENT APPLICATION

(11) **EP 0 535 440 A2**
(43) Date of publication of application: **07.04.1993**
(21) Application number: 92115757.4
(22) Date of filing: 15.09.1992
(51) Int. Cl.: A61K 31/205

(54) **The use of L-carnitine in endangered pregnancy**

(30) Priority: 18.09.1991 IT RM910695
(71) Applicant: Sigma-Tau Industrie Farmaceutiche Riunite S.p.A., I-00144 Roma (IT)
(72) Inventor: Cavazza, Claudio, I- Rome (IT)
(74) Representative: La Ciura, Salvatore

(57) **Abstract**

The administration of L-carnitine and pharmaceutically acceptable salts thereof for treatment of women susceptible to having an endangered pregnancy due to the presence of a hereditary defect in fatty acid metabolism, significantly improves the rate of survival of their offspring.

## Description

### BACKGROUND OF THE INVENTION

### Field of the Invention

The present invention relates to a new therapeutic use of L-carnitine. More specifically, the invention relates to the use of L-carnitine for the therapeutic treatment of pregnant women who have hereditary diseases associated with the metabolism of fatty acid.

### Discussion of the Background

Various studies have been conducted to determine variations in the blood levels of carnitine and endogenous acyl carnitines during the course of pregnancy. See for example C. Bargen-Lockner et al., Am. J. Obstet. Gynecol., 1981, 140:412; G. Cederblad, Acta. Paediatr. Scand., 1985, 74:500-504 and G. Cederblad et al, The American Journal of Clinical Nutrition, 1986, 44:379-383. These studies established that the blood level of free carnitine and total carnitine decreases during pregnancy.

During pregnancy, women who are pregnant for the first time (primiparous) or women having a repeat pregnancy (multiparous) may have an "endangered pregnancy"if the woman has a hereditary disease which affects the metabolism of fatty acids. In such women expressing these hereditary diseases, the defeat in fatty acid metabolism is transmitted to the offspring and presents a substantial risk to survival of the fetus and of the infant. Many infants born of mothers having hereditary defects in fatty acid metabolism die in utero or shortly after birth.

There is a continuing need, therefore, for methods of treating endangered pregnancy in order to increase the survival rate of a fetus or infant born to a woman having a hereditary disease associated with fatty acid metabolism.

### SUMMARY OF THE INVENTION

Accordingly, one object of the present invention is to provide a method of therapeutically treating endangered pregnancy.

This and other objects which will become apparent in the course of the following description of examplary embodiments have been achieved by the present use of L-carnitine or a salt thereof for treatement of endangered pregnancy.

### DETAILED DESCRIPTION OF THE PREFERRED EMBODIMENTS

As used herein, the term "endangered pregnancy"means a pregnancy in a woman who is subject to a hereditary disease which impairs fatty acid metabolism and can be transmitted to offspring of the woman. Any endangered pregnancy as defined above may be treated according to the method of the present invention. A defect in fatty acid metabolism is any defect which alters the normal metabolism of fatty acids in humans.

Hereditary diseases producing the endangered pregnancy which are treatable by the present invention include:
- Lipid storage myopathy
- Shot chain acyl-CoA dehydrogenase (SCAD) deficiency
- Medium chain acyl CoA-dehydrogenase (MCAD) deficiency
- Long chain acyl CoA-dehydrogenase (LCAD) deficiency
- Multiple acyl CoA-dehydrogenase (MAD) deficiency
- Isovaleric acidemia
- Propionic acidemia
- Methylmalonic aciduria
- Hydroxymethylglutaryl-CoA Iyase deficiency.

Glutaryl-CoA dehydrogenase deficiency (glutary aciduria type I)
- Beta-kethiolase deficiency
- Homocystinuria
- 5-methylene tetrahydrofolate reductase deficiency
- Adenosine deaminase deficiency
- Ornithine trancarbamoylase heterozygote state
- Cystinosis
- Citochrome oxidase deficiency.

The woman having an endangered pregnancy may be either multiparous or primiparous.

It is possible to determine whether a particular person is subject to an endangered pregnancy as defined above by means of routine case history investigation to determine whether previous children born alive have been subject to rapid death after birth or, whether intrauterine deaths and spontaneous abortions have occured. The routine case history is generally supplemented with laboratory tests which determine the amount or concentration of free carnitine and the relationship between acetyl carnitine and free carnitine in a biological sample. Typical biological samples tested for the presence of carnitine and acyl carnitine include blood, urine and organ tissue sample obtained by standard biopsy procedures. The amount and concentration of free carnitine and the relationship between acyl carnitine and free carnitine are determined using known methods, see for example c.p. Ceberblad et al, Clin.Chim., Acta, 1972, 37: 235-243; T. Bohemer et al, Clin.Chim., Acta, 1974, 57: 55-61; M.E. Mitchell Am. J. Clin. Nutr., 1978, 31: 481-491; L.L. Bieber et al, Methods in Enzymology, 1986, 123: 264-276; P. Harper et al, Eur. J. Clin. Pharmacol., 1988, 35: 69-75; C. de Sousa et al, Clin. Chim. Acta, 1990, 187: 317-328, and C.J. Rebouche, Metabolism. 1991, 40 (12): 1305-1310.

The normal adult non pregnant female values are 39 ± 9 and 5 ± 2 µ moles 1 respectively for free and esterified carnitine. In a pregnant woman, as pregnancy progresses, carnitine level decreases in a time frame similar to the increase in material blood volume. Both the free and esterified carnitine levels drop. However, there is an increase in the esterified to free ratio, with a relative decrease in free carnitine, the biologically active component. Allowing for individual variation, an esterified carnitine to free carnitine ratio exceeding 0,45 (normal) mean value 0,30) can be generally regarded as the critical threshold, in order to determine the risk of endangered pregnancy.

In the present method, L-carnitine or a pharmaceutically acceptable salt thereof is administered to a woman who is subject to a hereditary disease affecting the metabolism of fatty acids. L-carnitine or its pharmaceutically acceptable salts are preferably administered before the start of pregnancy in a woman who foresees becoming pregnant. Alternatively, the L-carnitine or its salt can be administered to a woman who is already pregnant, but administration should be started as soon as the woman becomes aware that she is in a stage of gestation and/or is suffering from the hereditary disease of lipid metabolism. Improved results are obtained if administration of L-carnitine or its pharmaceutically acceptable salt is begun prior to pregnancy and continued throughout pregnancy.

Pharmaceutically acceptable salts of L-carnitine include all pharmaceutically accetable salts which are prepared by the addition of acid to L-carnitine, and which do not give rise to undesirable toxic or collateral effects.

The formation of pharmaceutically acceptable acid addition salts is well known in pharmaceutical technology. Non-limiting example of suitable salts include the chloride, bromide, orotate, acid aspartate, acid citrate, acid phosphate, fumarate, acid fumarate, lactate, maleate, acid maleate, acid oxalate, acid sulfate, glucose phosphate, tartrate and acid tartrate salts. Other suitably acceptable salts which are non toxic and provide substantially similar results to administration of L-carnitine and the above-identified pharmaceuticals salts will be readily apparent to one having ordinary skill in the art and are considered to be equivalent to the salts enumerated above.

The L-carnitine may be administered orally or parenterally to a woman patient who is or foresees becoming pregnant in an amount sufficient to prevent intrauterine death and spontaneous abortion or death of the infant. The amount of L-carnitine or its pharmaceutically acceptable salt which is administered to a specific person will ordinarily be adjusted according to individual characteristics of each patient.

Generally, the amount of L-carnitine or its salt will be in the range of about 30-100 mg/kg/day, preferably about 50-70 mg/kg/day.

Obviously, amounts slightly above or below these general ranges may be administered so long as the desired prevention of intrauterine death, spontaneous abortion or infant death is attained. If desired, the L-carnitine or its salt may be incorporated into a conventional pharmaceutical carrier or excipient to facilitate administration.

By administering L-carnitine or a pharmaceutically acceptable salt thereof to a woman having a "endangered pregnancy", the method of the present invention substantially decreases the occurance of intrauterine death and spontaneous abortion. Additionally, the method of the present invention results in substantially greater numbers of surviving infants born to women having a hereditary defect in fatty acid metabolism. The present method, therefore, provides a very useful and important way to improve infant survival in women subject to hereditary fatty acid metabolism defects.

Other features of the invention will become apparent in the course of the following description of an exemplary embodiment which is given for illustration of the invention and is not intended to be limiting thereof.

### EXAMPLES

A clinical study was conducted on 70 pregnant women who in earlier pregnancies had given birth to infants who had died suddenly or who had suffered intrauterine death or spontaneous abortion of a fetus. All 70 of these patients demonstrated deficient serum levels of free carnitine both before pregnancy and during pregnancy and, therefore, were determined to have endangered pregnancies as a result of hereditary diseases producing defects in lipid metabolism.

Each pregnant woman was treated by administering 50-70mg kg/day of L-carnitine orally for a period time ranging from 3 months to 6 months prior to birth. The offspring were observed for one year after birth.

At the end of this study, a comparison was made between the earlier pregnancies which had occurred without administration of L-carnitine according to the present method and the most recent pregnancy with concurrent administration of L-carnitine in the amount indicated above. During the course of the previous pregnancies without co-administration of L-carnitine, the 70 wonen studies had 184 pregnancies with 186 potential offspring. The two additional potential offspring were the result of twin births in the women. Of these total 186 potential offspring, there were 67 cases of spontaneous abortions or intrauterine death (36%); 53 cases of sudden infant death (28,5%) and 66 cases of surviving infants (35,5%).

In the pregnancies studies during the present clinical study, the 70 women had 80 pregnancies with 81 offspring. The one additional child was the result of a single twin birth. All 81 infants (100%) survived to the end of the one-year study period.

The clinical study described above clearly indicates that administration of L-carnitine or a pharmaceutically acceptable salt thereof to a woman having an inheritable defect in lipid metabolism results in a surprising and substantial increase in the survival rate of infants born to these mothers.

Obviously, numerous modifications and variations of the present invention are possible in light of the above teachings. It is therefore to be understood that within the scope of the appended claims, the invention may be practiced otherwise than as specifically described herein.

## Claims

1. The use of L-carnitine and its pharmacologically acceptable salts to prepare a drug for endangered pregnancy.

2. The use of L-carnitine and its pharmacologically acceptable salts to prepare a drug to administer to human beings of the female sex, who are bearers of diseases of the metabolism of the fatty acids.

3. The use according to Claim 2 in which the said bearers of diseases of the metabolism of the fatty acids are pregnant.

4. The use according to Claim 3 in which the said pregnant women are primiparous.

5. The use according to Claim 3 in which the said pregnant women are multiparous.

6. The use of L-carnitine and its pharmaceutically acceptable salts for the treatment of endangered pregnancy which comprises the administration by the oral or parenteral route, to women who are bearers of diseases of the metabolism of the fatty acids, who are or foresee becoming pregnant, of 50-70mg/kg/day of L.carnitine or of an equivalent quantity of one of its pharmacologically acceptable salts.
